# EUROPEAN PATENT SPECIFICATION

(11) **EP 2 787 874 B1**
(45) Date of publication and mention of the grant of the patent: **18.07.2018**
(21) Application number: 12799172.7
(22) Date of filing: 08.12.2012
(51) Int. Cl.: A61B 1/008, A61M 25/01, A61B 1/00, A61B 1/005, A61B 5/06, G02B 23/24

(54) **INTRACORPOREAL LOCATOR PROBE**
INTRAKORPORALE LOKALISATORSONDE
SONDE DE LOCALISATION INTRACORPORELLE

(30) Priority: 08.12.2011 GB 201121064
(43) Date of publication of application: 15.10.2014
(73) Proprietor: Haemoband Surgical Limited, Belfast, Antrim BT6 8DD (GB)
(72) Inventor: GHAREEB, Essam, Ballinamllard Fermanagh BT94 2LS (GB)
(74) Representative: FRKelly
(86) International application number: PCT/EP2012/074876
(87) International publication number: WO 2013/083824

(56) References cited:
- JP-A- 2003 102 677
- JP-A- 2007 307 241
- US-A1- 2002 088 931
- US-A1- 2002 183 592
- US-A1- 2005 228 274
- US-A1- 2010 210 908
- US-A1- 2011 282 154

## Description

### Field of the Invention

The present invention relates to probes, especially probes for insertion into a human or animal body.

### Background to the Invention

Many investigative or treatment procedures involve the introduction of catheters, guide wires or scopes inside the arteries, veins, cavities or the gastrointestinal tract.

For the clinician to locate exactly the pathway of the instrument, X-Ray is needed in most cases in the form of fluoroscopy, e.g. in coronary angiography, or CT scan. Ultra sound can also be used to guide a needle into a specific location, e.g. when taking a biopsy from a tumour. In these procedures the patient, doctor and attendant are exposed to X-Rays. This limits its use to short bursts at intervals.

Endoscopists during colonoscopy are, in some units, provided by an imager. This device is partly incorporated into the scope to send signals to the receiver, which is placed in front of the patient. The signals are interpreted and shown on a screen delineating the shape of the scope. The device does not show the wall of the bowel but a clinician can deduce the position and shape of the scope in the bowels from the image available.

Introducing a central venous line is usually performed blindly or by using an ultra sound device. This device guides the clinician to the correct point of entry, but the progress of the catheter and its final position are conventionally determined by a plain film X-Ray. This is considered to be necessary in order to rule out any complication of a procedure, but if it shows an incorrect position of the line, the whole preparation and procedure has to be repeated.

In general, clinicians can attempt to determine the location of a device inside a body part without seeing the walls of the body part from their knowledge of anatomy.

Disadvantages of the above systems and techniques include: the need for specially built and equipped rooms to prevent the dissemination of X-Rays; the involvement of more than one discipline in the procedure, which limits availability; exposure to X-Rays; in some cases the complete absence of a system to help guide the clinician in the procedure; the expense of the necessary equipment.

It would be desirable to mitigate at least some of these problems.

US patent application US2005228274 discloses a catheter device with bending sensors. Japanese patent applications JP2003102677 and JP2007307241 each discloses an endoscope apparatus with bend sensors. US patent applications US2010210908 and US2002183592 each discloses an endoscope with bend detection capabilities. US patent application US2002088931 discloses a topological and motion measuring tool with flexure sensors. US patent application US2011282154 discloses a medical apparatus with bend detection capabilities

### Summary of the Invention

A first aspect of the invention provides a probe monitoring system as claimed in claim 1.

Preferably, the probe device comprises at least one set of bend sensors, the or each set comprising at least two bend sensors that are substantially in register with one another longitudinally of said body and mutually spaced apart around said body. More preferably, there is a plurality of said sets of bend sensors, mutually spaced apart along the length of said body. Advantageously, said at least two bend sensors of the or each set are positioned to detect bending of said body in a respective one of two mutually orthogonal planes.

Typically, said at least one bend sensor comprises a sensing component formed from a bend-sensitive material, for example a piezoresistive material, carbon or a conductive fabric.

Preferably, the system further includes processing means configured to superimpose an image of said probe device, generated from said data model, with an image of an object, for example part of a human or animal body, into which the probe device is inserted during use.

Other preferred features are recited in the dependent claims.

Further advantageous aspects of the invention will be apparent to those ordinarily skilled in the art upon review of the following description of preferred embodiments and with reference to the accompanying drawing.

### Brief Description of the Drawings

An embodiment of the invention is now described by way of example and with reference to the accompanying drawings in which
Figure 1A is a schematic view of a probe device embodying one aspect of the invention included in a probe monitoring system embodying another aspect of the invention;
Figure 1B is a cross-sectional end view of the probe device of Figure 1A;
Figure 2 shows schematic representations of a bend sensing component; and
Figure 3 shows a bend sensor and associated electrical circuit.

### Detailed Description of the Drawings

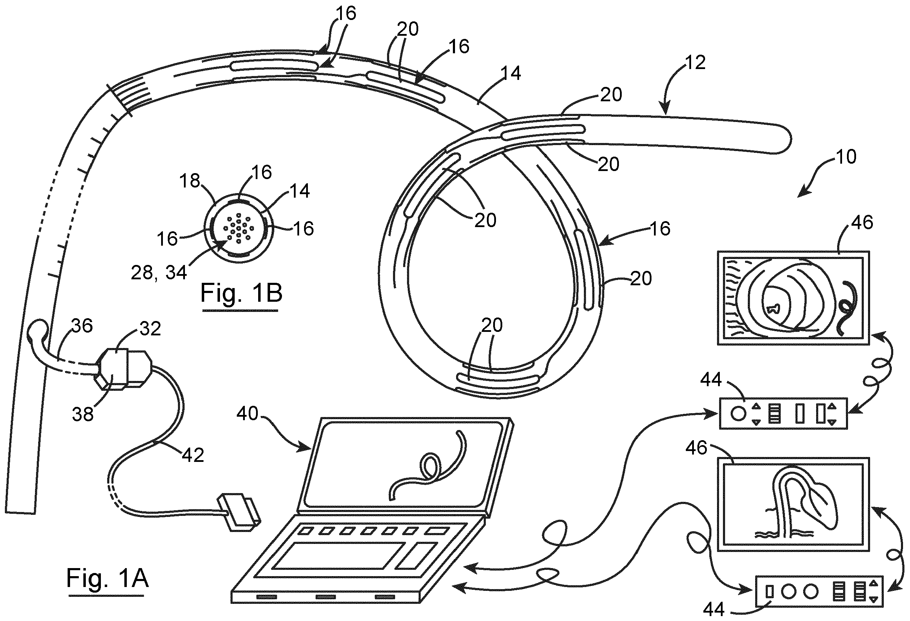

Referring now to the drawings, there is shown generally indicated as 10 a probe monitoring system embodying one aspect of the invention. The system 10 includes a probe device 12 embodying another aspect of the invention. The probe device 12 comprises an elongate flexible body 14, which may be formed from any suitable material typically plastics or a dielectric material. The body 14 may have any cross-sectional shape although it is typically substantially circular in cross-section, as shown by way of example in Figure 1B. Alternatively, the body 14 may comprise a substrate having a substantially rectangular, or otherwise polygonal, cross-section defining substantially flat surfaces on each face of the body 14. The body 14 is flexible (and preferably non-resilient) advantageously to the extent that it can bend freely during use to conform to the shape of any passage into which it is introduced. For example, the body 14 may be made of a material that allows it to bend in two mutually perpendicular planes, or to bend in a first plane and to be rotatable (or twistable) about an axis in that plane to allow it to bend in a plane that is perpendicular to the first plane.

In some embodiments, the probe device 12 may be intended for use as a catheter, in which case the body 14 may be hollow to allow fluids to pass through it or to allow another probe (not shown), for example an endoscope or other instrument, to be fed through it. In other embodiments, the probe device 12 may itself be intended for use as an endoscope or other instrument, in which case the body 14 may be solid or hollow as required by the application and may incorporate other devices, e.g. a camera and/or a lamp (not shown) as required. Alternatively still, the probe device 12 may be located, in use, in a channel or passage formed in another probe, e.g. an endoscope or other instrument.

A plurality of bend sensors 16 are provided on the body 14 in order to detect bends in the body 14. In the illustrated embodiment, each bend sensor 16 is provided on an outer surface of the body 14, preferably covered by one or more sheath 18 which is fitted over the body 14 and may extend wholly or partly along the length of the body 14, preferably at least being long enough to cover the sensors 16. The sheath 18 may be formed from any suitable material, e.g. rubber or plastics. The sensors 16 may be fixed in position by any suitable means, e.g. adhesive. Alternatively, the sensors 16 may be embedded in the body 14, or provided on an internal surface of the body 14.

The device 12 includes means for producing an output signal that is indicative of at least one characteristic of a detected bend the body. Typically this is provided by an electrical circuit forming part of the bend sensor and/or to which the bend sensor 16 is connected. In the illustrated embodiment, each bend sensor 16, when incorporated into a suitable electrical circuit, causes the circuit to generate an electrical output signal that is indicative of one or more characteristics of a bend in the portion of the body 14 at which the sensor 16 is located. For example, the electrical output signal may be indicative of the extent of the bend (i.e. the relative sharpness of the bend) and/or the direction of the bend (i.e. whether it is convex or converse with respect to a notional reference point). One or more thresholds may be defined for determining when a bend is part of a loop. For example, the extent of a bend (i.e. the relative sharpness of the bend) as determined from the output signal may be compared against one or more threshold value, and depending on the result, e.g. if a relevant threshold is exceeded, then the presence of a loop is determined.

In preferred embodiments, each bend sensor 16 comprises a sensing component 20 having an electrical property, conveniently electrical resistance, that changes depending on how the sensing component is bent. The sensing component 20 is incorporated into an electrical circuit that produces an output signal that is dependent on the value of the resistance, or other relevant electrical property, of the sensing component 20. Advantageously, the circuit is configured such that the output signal continuously indicates the extent to which the component 20 is bent, i.e. is continuously indicative of the relevant electrical property, typically resistance, of the component 20. The sensing component 20 typically comprises an elongate strip and may comprise any suitable bend-sensitive material, for example a piezoresistive material (typically semiconductor materials such as germanium, polycrystalline silicon, amorphous silicon, silicon carbide, or single crystal silicon), carbon or a conductive fabric or a conductive or resistive ink (any of which may provided on or in a substrate if necessary, e.g. on a surface, especially a flat surface, of the body 14). Optionally, each sensing component 20 may comprise two or more sensing components, of any suitable type including those described above, electrically connected in series. Optionally, where the sensing component 20 comprises a strip of bend-sensitive material, one or more electrically conductive pads may be provided along its length (spaced-apart where there is more than one) in order to control, and typically reduce, the electrical resistance of the strip.

Typically, each sensing component 20 has an axis along which it is sensitive to being bent (i.e to produce the change in electrical property in this example). Where the sensing component 20 is elongate, the bend-sensitive axis is typically the longitudinal axis. In use, the sensing element 20 is therefore capable of detecting bending of the object on which it is located in a plane in which the bend-sensitive axis lies.

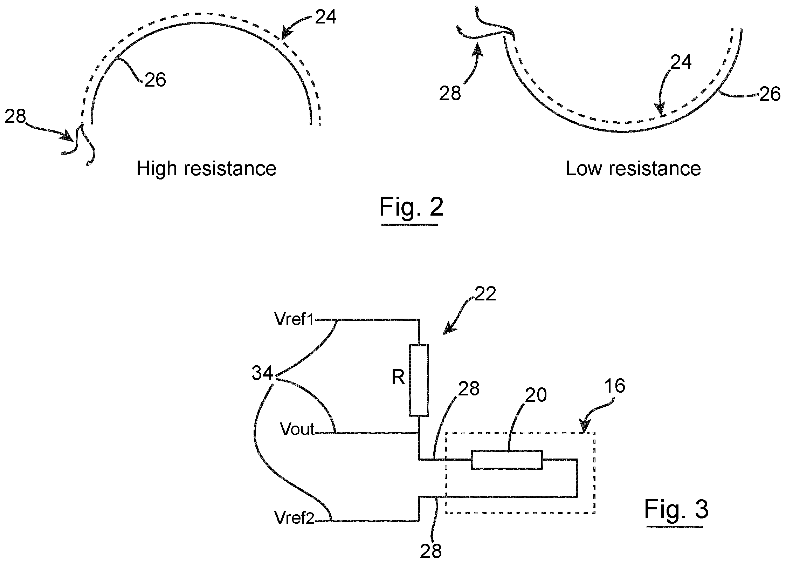

Figures 2 and 3 show an example of a bend sensor 16 comprising a sensing component 20 incorporated into an electrical circuit 22 in the form of a voltage divider circuit. By way of example only, the sensing component 20 comprises carbon elements 24 on a substrate 26. When the sensing component 20 bends in one direction (convex or concave with respect to a notional reference point) its electrical resistance increases in proportion to the severity of the bend, and when it bends in the other direction (concave or convex with respect to the notional reference point) its electrical resistance decreases in proportion to the severity of the bend. Hence, the voltage level at the output Vout depends on the relative resistances of resistor R and the sensing component 20 and will tend towards reference value Vref1 as the resistance of the sensing component 20 increases or towards reference value Vref2 as the resistance of the sensing component 20 decreases.

Conveniently, the respective sensing components 20 are located on the body 14 (e.g. mounted on its surface or embedded therein), and are electrically connected to the respective electrical circuit by (typically a pair of) conductive wires 28, which may take the form of conductive tracks provided on a substrate (e.g. the body 14). In some embodiments, the respective electrical circuits are located remotely from the sensing components 20, for example at the proximal end of the probe 12 or separately from the probe 12, e.g. in a signal processing device 32. Alternatively, the respective electrical circuits may be co-located with the sensing components 20 on the body 14. In either case, electrical wires (which may comprise the wires 28 or the wires 34 (either of which may take the form of conductive tracks provided on a substrate, e.g. the body 14) depending on where the respective circuits are located) may be carried by the surface of the body 14, e.g. between the body 14 and the sheath 18 when present, or may be fed through the inside of the body 14 (whether the body is solid or hollow) as is convenient to the application. If the body is solid, one or more passages may be formed for the wires, or the wires may be embedded in the solid core of the body. Typically, the wires are gathered in a cable 36 having a connector 38 (which may include suitable signal processing circuitry in some embodiments) to allow connection to other components of the system 10 as described in more detail hereinafter. In cases where the wires 28, 34 comprise conductive tracks, any suitable connector (not shown) may be provided for allowing connection to other components of the system, e.g. a connector allowing connection of the tracks to the cable 36.

In alternative embodiments the sensing components 20/sensors 16 on the body 14 may be equipped to communicate wirelessly with the monitoring system 10.

The sensors 16 (and more particularly the respective sensing components 20) are spaced apart along the length of the body 14 (i.e. longitudinally spaced along the body 14). As such, each sensor 16 detects bends in the portion of the body 14 at which it is located. Preferably, the respective bend-sensitive axis of the respective sensing components 20 are substantially aligned with the longitudinal axis of the body 14. In preferred embodiments, the sensors 16 (and more particularly the respective sensing components 20) are arranged in sets of at least two, the sets being spaced apart along the length of the body 14, each sensing component 20 of each set preferably being substantially in register with the other longitudinally of the body 14, but spaced apart around the body 14 (i.e. transversely spaced around the body). Advantageously, at least two of the sensing components 20 in each set are mutually spaced apart around the body 14 such that they are capable of detecting bending of the body 14 in respective mutually orthogonal planes. For example, the respective sensing components 20 may be radially spaced around the body by 90° or substantially 90°.

In one embodiment, therefore, each set comprises two sensors 16 mutually spaced apart around the body 14 and positioned to detect bending of the body 14 in respective mutually orthogonal planes. This embodiment is particularly suited to using sensing components 20 of the general type shown in Figure 2 which are capable of distinguishing between convex and concave bends in a given plane. Other types of sensing components 20 may only be capable of detecting either convex or concave bends in a given plane. In such cases, it is preferred that each set comprises at least four sensors arranged in pairs, each pair being arranged to detect bending in a mutually orthogonal plane, the sensing components within each pair being arranged to detect bending in opposite senses (concave or convex) in the respective plane. To this end, the sensing components of each pair may be substantially oppositely disposed on the body 14. In the illustrated embodiment, each set comprises four sensors 16 equally spaced around the body 14. In cases where the sensing element 20 comprises two or more individual sensing elements connected in series, the individual elements may be aligned end-to-end or side-by-side in order that they are subjected to bending in the same sense, or they may be spaced-apart around the periphery of the body 14 such that they are subjected to bending in respective opposite senses.

In any event, the preferred arrangement is such that each set of sensors 16 is capable of detecting bending of the body 14 in two mutually orthogonal planes, i.e. two orthogonal planes that are each orthogonal to the body's transverse plane at the respective location along the body 14. As a result, the signals received from each set of sensors 16 can be used to build a three dimensional image of the probe 12, including any bends or loops that may be formed during use. The longitudinal spacing between adjacent sets affects the resolution of the probe image that can be built and may be selected to suit the application.

The system 10 comprises a monitoring device 40 that includes means for processing the signals received from the sensors 16 in order to create a data model representing the shape of the probe 12 in three dimensions. This may be achieved combining the respective output signals from the sensors 16 of each set with an indication of each set's longitudinal location on the body 14. This may be achieved by appropriate configuration of the connector 38/signal processor 32 as an interface between the sensors 16 and the monitor 40. The device 40 preferably also includes means for rendering the data model as an image on a VDU, which may be part of or otherwise connected to the monitoring device 40. The device 40 conveniently comprises a computer running suitable computer software for creating the data model and preferably also rendering the image. The data model may be created using any convenient conventional techniques and may be configured to use the bend information determined from the output signals generated by the sensors 16. Loops in the probe 12 may be identified by comparing the relative sharpness of one or more detected bends against one or more threshold values. The respective outputs from more than one sensor 16 may be analysed, e.g. aggregated or otherwise collectively assessed, to determine on which side the probe 12 crosses itself in a detected loop.

The connector 38/signal processor 32 may include analogue to digital conversion circuitry. This facilitates connection of the cable 36 to the monitoring device 40, e.g. by cable 42. The cable 36 (and optionally also the cable 42 in some embodiments) may be configured to deliver electrical power to the sensors 16, if required, the power being supplied by the monitoring device 40 for example. Alternatively, a separate electrical power source (not shown) may be provided for the probe 12, typically being connectable to the cable 36 or otherwise to the wires 36.

In preferred embodiments, the system 10 includes processing means for superimposing an image generated from the data model of the probe 12 with an image of the object, e.g. human or animal body or part thereof, into which the probe 12 is inserted. Typically, this is achieved by suitable computer software running on a computer. This enables a user to see where the probe 12 is in relation to the object. The image of the object can be created by any suitable conventional means, e.g. a computed tomography (CT) scan, or magnetic resonance imaging (MRI) scan, and supplied to the superimposing processing means in any convenient manner. The superimposing software may be supported by the monitoring device 40 or may be supported elsewhere, e.g. by a separate video or image processing device 44 (which may have its own VDU 46). The monitoring device 40 and any other processor 44 may co-operate as necessary to display the superimposed image on one or more VDUs 44, 46 as applicable.

It is envisaged that preferred embodiments of the invention could be used in at least the following applications: Endoscopy (e.g. colonoscopy, small intestinal endoscopy, uretroscopy or nasojujenal tubes); Anaesthesia/ICU (e.g. central venous line insertion, Heckman lines insertion, PICC lines insertion, tracheal intubation or dialysis catheters); Cardiology (e.g. coronary angioplasty (superimposed on CT scan pictures or other images), pace maker introduction, pericardiocentesis or intra-aortic balloon pumps); Vascular procedures (e.g. angioplasty, EVARs, embolectomy catheters; Urology (e.g. urinary catheter introduction); or Thoracic surgery (e.g. chest drain positioning).

Preferred embodiments of the invention are capable of recognising bending or looping inside an object without the need for any other detectors, field generators or X-ray devices outside of the object. The preferred system is operable independently of other equipment and preferably has its own power supply. The preferred system does not generate significant electromagnetic fields that may interfere with other devices, e.g. cardiac pace-makers.

The invention is not limited to the embodiment described herein which may be modified or varied without departing from the scope of the invention.

## Claims

1. A probe monitoring system (10) comprising:
a probe device (12) comprising an elongate flexible body (14), a plurality of bend sensors (16) located at respective portions of the body spaced apart along the length of the body, said bend sensors being configured to detect bending of the respective portion of the body, and means (22) for producing a respective output signal for each bend sensor that is indicative of at least one characteristic of a detected bend in the respective portion of the body; and
processing means (40) configured to create a data model of said probe device from the respective output signals of said bend sensors,
**characterised in that** said at least one characteristic comprises a relative sharpness of the detected bend and **in that** said processing means is configured to detect loops in the probe device by comparing the relative sharpness of one or more detected bends against one or more threshold values, and if a relevant threshold is exceeded, then the presence of a loop is determined.

2. The probe monitoring system as claimed in claim 1, comprising at least one set of bend sensors (16), the or each set comprising at least two bend sensors that are substantially in register with one another longitudinally of said body and mutually spaced apart around said body (14), said at least one set of bend sensors preferably comprising a plurality of said sets of bend sensors, mutually spaced apart along the length of said body.

3. The probe monitoring system as claimed in claim 2, wherein said at least two bend sensors (16) of the or each set are positioned to detect bending of said body (14) in a respective one of two mutually orthogonal planes, and wherein, preferably, said at least two bend sensors are radially spaced apart around said body by substantially 90°.

4. The probe monitoring system as claimed in any preceding claim, wherein each bend sensor (16) is differently responsive to bending of said body (14) in opposite senses.

5. The probe monitoring system as claimed in any preceding claim, wherein each bend sensor (16) is similarly responsive to bending of said body (14) in opposite senses.

6. The probe monitoring system as claimed in claim 5, when dependent on any one of claims 2 to 4, wherein the or each set comprises one or more pairs of oppositely disposed bend sensors (16).

7. The probe monitoring system as claimed in any preceding claim, wherein each bend sensor (16) exhibits an electrical characteristic, for example electrical resistance, that changes depending on at least one characteristic of a bend to which the bend sensor is subjected, wherein said at least one characteristic includes the sharpness of the bend and/or the sense of the bend.

8. The probe monitoring system as claimed in any preceding claim wherein said output signal producing means (22) comprises an electrical circuit configured to produce an output signal that is indicative of at least one characteristic of a bend in the respective bend sensor (16).

9. The probe monitoring system as claimed in any preceding claim, wherein said output signal producing means (22) comprises a respective electrical circuit for each of said bend sensors (16), the or each electrical circuit being configured to produce an output signal that is indicative of at least one characteristic of a bend in the respective bend sensor.

10. The probe monitoring system as claimed in claim 8 or 9, wherein the respective electrical circuit (22) is part of the respective bend sensor (16), or wherein the respective bend senor is connected to said electrical circuit, preferably to a respective electrical circuit.

11. The probe monitoring system as claimed in any preceding claim, wherein each bend sensor (16) comprises a sensing component formed from a bend-sensitive material, for example a piezoresistive material, carbon, a conductive fabric or a conductive or resistive ink, and wherein said sensing component preferably comprises an elongate strip.

12. The probe monitoring system as claimed in any preceding claim, further comprising rendering means (40) configured to render said data model as an image of said probe device (12), and wherein, preferably, said processing means (40) is configured to superimpose an image of said probe device, generated from said data model, with an image of an object, for example part of a human or animal body, into which the probe device is inserted during use.

13. The probe monitoring system as claimed in claim 1, wherein said processing means (40) is configured to determine on which side the probe device (12) crosses itself in a detected loop by analyzing the respective outputs from more than one bend sensor (16).

## Patentansprüche

1. Sondenüberwachungssystem (10), das Folgendes umfasst:
eine Sondenvorrichtung (12), die Folgendes umfasst: einen länglichen flexiblen Körper (14), eine Vielzahl von Biegesensoren (16), die an jeweiligen Teilen des Körpers angeordnet sind, die entlang der Länge des Körpers voneinander beabstandet sind, wobei die Biegesensoren dafür konfiguriert sind, das Biegen des jeweiligen Abschnitts des Körpers zu erfassen, und ein Mittel (22) zum Erzeugen eines entsprechenden Ausgangssignals für jeden Biegesensor, das mindestens eine Eigenschaft einer erfassten Biegung in dem jeweiligen Abschnitt des Körpers anzeigt; und
ein Verarbeitungsmittel (40), konfiguriert für das Erzeugen eines Datenmodells der Sondenvorrichtung aus den jeweiligen Ausgangssignalen der Biegesensoren,
**dadurch gekennzeichnet, dass** die mindestens eine Eigenschaft eine relative Schärfe der erfassten Biegung umfasst und dass das Verarbeitungsmittel dafür konfiguriert ist, Schleifen in der Sondenvorrichtung durch Vergleichen der relativen Schärfe einer oder mehrerer erfasster Biegungen mit einem oder mehreren Schwellenwerten zu erfassen, und wenn ein relevanter Schwellenwert überschritten wird, das Vorhandensein einer Schleife festgestellt wird.

2. Sondenüberwachungssystem nach Anspruch 1, das mindestens einen Satz von Biegesensoren (16) umfasst, wobei der oder jeder Satz mindestens zwei Biegesensoren umfasst, die im Wesentlichen in Längsrichtung des Körpers zueinander ausgerichtet und voneinander beabstandet um den Körper (14) angeordnet sind, wobei der mindestens eine Satz von Biegesensoren vorzugsweise eine Vielzahl der Sätze von Biegesensoren umfasst, die entlang der Länge des Körpers voneinander beabstandet angeordnet sind.

3. Sondenüberwachungssystem nach Anspruch 2, wobei die mindestens zwei Biegesensoren (16) des oder jedes Satzes dafür angeordnet sind, das Biegen des Körpers (14) in einer jeweiligen von zwei zueinander orthogonalen Ebenen zu erfassen, und wobei die mindestens zwei Biegesensoren vorzugsweise radial um den Körper herum im Wesentlichen um 90 ° beabstandet sind.

4. Sondenüberwachungssystem nach einem der vorhergehenden Ansprüche, wobei jeder Biegesensor (16) unterschiedlich auf das Biegen des Körpers (14) in entgegengesetzten Richtungen anspricht.

5. Sondenüberwachungssystem nach einem der vorhergehenden Ansprüche, wobei jeder Biegesensor (16) in ähnlicher Weise auf das Biegen des Körpers (14) in entgegengesetzten Richtungen anspricht.

6. Sondenüberwachungssystem nach Anspruch 5, wenn abhängig von einem der Ansprüche 2 bis 4, wobei der oder jeder Satz ein oder mehrere Paare gegenüberliegender Biegesensoren (16) umfasst.

7. Sondenüberwachungssystem nach einem der vorhergehenden Ansprüche, wobei jeder Biegesensor (16) eine elektrische Eigenschaft aufweist, beispielsweise einen elektrischen Widerstand, der sich abhängig von mindestens einer Eigenschaft einer Biegung ändert, der der Biegesensor ausgesetzt ist, wobei die mindestens eine Eigenschaft die Schärfe der Biegung und/oder die Richtung der Biegung umfasst.

8. Sondenüberwachungssystem nach einem der vorhergehenden Ansprüche, wobei das Ausgabesignal-Erzeugungsmittel (22) eine elektrische Schaltung aufweist, die dafür konfiguriert ist, ein Ausgabesignal zu erzeugen, das mindestens eine Eigenschaft einer Biegung in dem jeweiligen Biegesensor (16) anzeigt.

9. Sondenüberwachungssystem nach einem der vorhergehenden Ansprüche, wobei das Ausgangssignal-Erzeugungsmittel (22) eine jeweilige elektrische Schaltung für jeden der Biegesensoren (16) aufweist, wobei die oder jede elektrische Schaltung dafür konfiguriert ist, ein Ausgangssignal zu erzeugen, das mindestens eine Eigenschaft einer Biegung in dem jeweiligen Biegesensor anzeigt.

10. Sondenüberwachungssystem nach Anspruch 8 oder 9, wobei die jeweilige elektrische Schaltung (22) Teil des jeweiligen Biegesensors (16) ist, oder wobei der jeweilige Biegesensor mit der elektrischen Schaltung, vorzugsweise mit einer jeweiligen elektrischen Schaltung, verbunden ist.

11. Sondenüberwachungssystem nach einem der vorhergehenden Ansprüche, wobei jeder Biegesensor (16) eine Erfassungskomponente umfasst, die aus einem biegeempfindlichen Material, beispielsweise einem piezoresistiven Material, Kohlenstoff, einem leitfähigen Gewebe oder einer leitfähigen oder resistiven Tinte gebildet ist, und wobei die Sensorkomponente vorzugsweise einen länglichen Streifen umfasst.

12. Sondenüberwachungssystem nach einem der vorhergehenden Ansprüche, das ferner ein Rendermittel (40) umfasst, das dafür konfiguriert ist, das Datenmodell als Bild der Sondenvorrichtung (12) zu rendern, und wobei das Verarbeitungsmittel (40) vorzugsweise dafür konfiguriert ist, ein Bild der Sondenvorrichtung, das aus dem Datenmodell erzeugt wird, mit einem Bild eines Objekts, beispielsweise eines Teils eines menschlichen oder tierischen Körpers, in den die Sondenvorrichtung während des Gebrauchs eingesetzt wird, zu überlagern.

13. Sondenüberwachungssystem nach Anspruch 1, wobei das Verarbeitungsmittel (40) dafür konfiguriert ist, zu bestimmen, auf welcher Seite sich die Sondenvorrichtung (12) in einer erkannten Schleife kreuzt, indem die jeweiligen Ausgaben von mehr als einem Biegesensor (16) analysiert werden.

## Revendications

1. Système de surveillance à sonde (10) comprenant :
un dispositif de sonde (12) comprenant un corps souple allongé (14), une pluralité de capteurs de flexion (16) localisée au niveau de portions respectives du corps espacées le long de la longueur du corps, lesdits capteurs de flexion étant configurés pour détecter une flexion de la portion respective du corps, et un moyen (22) pour une production d'un signal de sortie respectif pour chaque capteur de flexion qui est indicateur d'au moins une caractéristique d'une flexion détectée dans la portion respective du corps ; et
un moyen de traitement (40) configuré pour créer un modèle de données dudit dispositif de sonde à partir des signaux de sortie respectifs desdits capteurs de flexion, **caractérisé en ce que** ladite au moins une caractéristique comprend une importance relative de la flexion détectée et **en ce que** ledit moyen de traitement est configuré pour détecter des boucles dans le dispositif de sonde en comparant l'importance relative d'une ou de plusieurs flexions détectées par rapport à une ou plusieurs valeurs de seuil, et si un seuil pertinent est dépassé, alors la présence d'une boucle est déterminée.

2. Système de surveillance à sonde selon la revendication 1, comprenant au moins un ensemble de capteurs de flexion (16), le ou chaque ensemble comprenant au moins deux capteurs de flexion qui sont sensiblement en alignement l'un avec l'autre de manière longitudinale dudit corps et mutuellement espacés autour dudit corps (14), ledit au moins un ensemble de capteurs de flexion comprenant de préférence une pluralité desdits ensembles de capteurs de flexion, mutuellement espacés le long de la longueur dudit corps.

3. Système de surveillance à sonde selon la revendication 2, dans lequel lesdits au moins deux capteurs de flexion (16) du ou de chaque ensemble sont positionnés pour détecter une flexion dudit corps (14) dans un respectif de deux plans mutuellement orthogonaux, et dans lequel, de préférence, lesdits au moins deux capteurs de flexion sont espacés de manière radiale autour dudit corps de sensiblement 90°.

4. Système de surveillance à sonde selon l'une quelconque revendication précédente, dans lequel chaque capteur de flexion (16) répond de manière différente à une flexion dudit corps (14) en sens opposés.

5. Système de surveillance à sonde selon l'une quelconque revendication précédente, dans lequel chaque capteur de flexion (16) répond de manière similaire à une flexion dudit corps (14) en sens opposés.

6. Système de surveillance à sonde selon la revendication 5, lorsque dépendant d'une quelconque des revendications 2 à 4, dans lequel le ou chaque ensemble comprend une ou plusieurs paires de capteurs de flexion (16) disposés de manière opposée.

7. Système de surveillance à sonde selon l'une quelconque revendication précédente, dans lequel chaque capteur de flexion (16) présente une caractéristique électrique, par exemple une résistance électrique, qui change en fonction d'au moins une caractéristique d'une flexion à laquelle le capteur de flexion est soumis, dans lequel ladite au moins une caractéristique inclut l'importance de la flexion et/ou le sens de la flexion.

8. Système de surveillance à sonde selon l'une quelconque revendication précédente dans lequel ledit moyen de production de signal de sortie (22) comprend un circuit électrique configuré pour produire un signal de sortie qui est indicateur d'au moins une caractéristique d'une flexion dans le capteur de flexion (16) respectif.

9. Système de surveillance à sonde selon l'une quelconque revendication précédente, dans lequel ledit moyen de production de signal de sortie (22) comprend un circuit électrique respectif pour chacun desdits capteurs de flexion (16), le ou chaque circuit électrique étant configuré pour produire un signal de sortie qui est indicateur d'au moins une caractéristique d'une flexion dans le capteur de flexion respectif.

10. Système de surveillance à sonde selon la revendication 8 ou 9, dans lequel le circuit électrique (22) respectif est une partie du capteur de flexion (16) respectif, ou dans lequel le capteur de flexion respectif est connecté audit circuit électrique, de préférence à un circuit électrique respectif.

11. Système de surveillance à sonde selon l'une quelconque revendication précédente, dans lequel chaque capteur de flexion (16) comprend un composant de détection formé à partir d'un matériau sensible à la flexion, par exemple un matériau piézorésistif, du carbone, un tissu conducteur ou une encre conductrice ou résistive, et dans lequel ledit composant de détection comprend de préférence une bande allongée.

12. Système de surveillance à sonde selon l'une quelconque revendication précédente, comprenant en outre un moyen de rendu (40) configuré pour rendre ledit modèle de données comme une image dudit dispositif de sonde (12), et dans lequel, de préférence, ledit moyen de traitement (40) est configuré pour superposer une image dudit dispositif de sonde, générée à partir dudit modèle de données, avec une image d'un objet, par exemple une partie d'un corps humain ou animal, dans lequel le dispositif de sonde est inséré pendant une utilisation.

13. Système de surveillance à sonde selon la revendication 1, dans lequel ledit moyen de traitement (40) est configuré pour déterminer sur quel côté le dispositif de sonde (12) se croise lui-même dans une boucle détectée par une analyse des sorties respectives à partir de plus d'un capteur de flexion (16).
